# EUROPÄISCHE PATENTSCHRIFT

(11) **EP 1 027 669 B1**
(45) Veröffentlichungstag und Bekanntmachung des Hinweises auf die Patenterteilung: **31.10.2001**
(21) Anmeldenummer: 98951173.8
(22) Anmeldetag: 14.08.1998
(51) Int. Cl.: G06F 17/30

(54) **VERFAHREN ZUR EINGRUPPIERUNG VON SEQUENZEN IN FAMILIEN**
METHOD FOR CLUSTERING SEQUENCES IN GROUPS
PROCEDE DE REGROUPEMENT DE SEQUENCES PAR FAMILLES

(30) Priorität: 17.10.1997 DE 19745665
(43) Veröffentlichungstag der Anmeldung: 16.08.2000
(73) Patentinhaber: Deutsches Krebsforschungszentrum Stiftung des öffentlichen Rechts, 69120 Heidelberg (DE)
(72) Erfinder: VINGRON, Martin, D-69123 Heidelberg (DE); KRAUSE, Antje, D-69120 Heidelberg (DE)
(74) Vertreter: Castell, Klaus, Dr.
(86) Internationale Anmeldenummer: DE9802422
(87) Internationale Veröffentlichungsnummer: WO9921107

(56) Entgegenhaltungen:
- HARRIS N L ET AL: "ClassX: a browsing tool for protein sequence megaclassifications" PROCEEDING OF THE TWENTY-SIXTH HAWAII INTERNATIONAL CONFERENCE ON SYSTEM SCIENCES (CAT. NO.93TH0501-7), WAILEA, HI, USA, 5-8 JAN. 1993, Seiten 554-563 vol.1, XP002091600 ISBN 0-8186-3230-5, 1993, Los Alamitos, CA, USA, IEEE, USA
- HARRIS N L ET AL: "Mega-classification: discovering motifs in massive datastreams" AAAI-92. PROCEEDINGS TENTH NATIONAL CONFERENCE ON ARTIFICIAL INTELLIGENCE, SAN JOSE, CA, USA, 12-16 JULY 1992, Seiten 837-842, XP002091665 1992, Menlo Park, CA, USA, AAAI Press, USA

## Beschreibung

Die Erfindung betrifft ein Verfahren zur Eingruppierung von Sequenzen in Familien.

In der Molekularbiologie werden heute große Mengen von Proteinsequenzdaten erzeugt. Hierbei ist ein großes Problem die sinnvolle Eingruppierung dieser Proteinsequenzdaten in biologische Familien. Da Familien nicht exakt definiert sind, sondern die Diversität verschiedener Genfamilien unterschiedlich ist, handelt es sich hierbei um ein nicht-triviales Problem der Datengruppierung.

Bisher konnte die Bioinformatik nur Hilfestellung für menschliche Experten leisten, die die Ausgabe von Datenbanksuchprogrammen durchforsten und eine Gruppierung nach Familien erstellen. Dieses Verfahren ist zeitaufwendig, arbeitsaufwendig und schlecht reproduzierbar.

Der Erfindung liegt daher die Aufgabe zugrunde, ein Verfahren zu finden, mit dem ein großer Satz von Proteinsequenzen vollautomatisch in Gruppen aufgeteilt wird.

Diese Aufgabe wird mit den Merkmalen des Patentanspruchs 1 gelöst.

Dem beschriebenen Verfahren liegt die Erkenntnis zugrunde, daß eine schnelle Eingruppierung erreicht werden kann, wenn traditionelle Datenbanksuchprogramme iterativ aufgerufen werden, um zu einer gegebenen Proteinsequenz eine Menge verwandter Sequenzen zu finden.

Vorteilhaft ist es, wenn das beschriebene Verfahren für jede Sequenz der Datenbank durchgeführt wird, die mehrfach auftretenden Cluster bis auf jeweils einen Cluster entfernt werden, Cluster, die in anderen Clustern enthalten sind, entfernt werden, von der verbleibenden Clustermenge, die Cluster, die nicht mit anderen Clustern überlappen als Partitionierung der Datenbank ausgegeben werden und der verbleibende Teil der überlappenden Cluster als Gruppen ausgegeben wird, deren Cluster untereinander durch Überlappungen verbunden sind.

Dadurch wird eine Datenbank-Clusterung erzielt, die zur Folge hat, daß der Großteil der Cluster zueinander disjunkt ist und daher eine gültige, sinnvolle Clusterung der Daten darstellt.

Dieses Verfahren ermöglicht eine wesentlich raschere und objektivere Analyse neuer Sequenzdaten als es bisher möglich war. Der paarweise disjunkte Teil der Clusterung bedarf praktisch keiner Überprüfung mehr und ist die ideale Basis für automatische Annotationen und weiterführende Analysen. Das Residuum, d. h. der verbleibende Teil der überlappenden Cluster ist der Teil, der von menschlichen Experten studiert werden muß. Dieser Teil ist extrem reduziert und zudem auch durch die überlappenden Cluster vorstrukturiert.

Das Verfahren ist in einer extrem kurzen Rechenzeit durchzuführen, da für die Clusterung einer ganzen Datenbank nicht mehr jede Sequenz seperat mit jeder anderen verglichen werden muß.

In einer vorteilhaften Ausführungsvariante liegt der Schwellenwert zwischen 10⁻²⁰ und 10⁻³⁵. In der Praxis hat sich ein Wert von 10⁻³⁰ bewährt. Das Verfahren ist außer zur Clusterung von Proteinsequenzen für DNA-Sequenzen geeignet. Hierbei kann eine Relaxierung des Schwellenwertes über 10⁻²⁰ hinaus sinnvoll sein.

Eine Weiterentwicklung des erfindungsgemäßen Verfahrens besteht darin, daß aus der Positivmenge der in einem Iterationsschritt gefundenen Sequenzen nicht nur die am schlechtesten bewertete Sequenz für eine weitere Datenbanksuche verwendet wird, sondern alle Sequenzen dieser Menge in weiteren Suchen als Suchsequenz dienen. Durch die größere Anzahl der durchzuführenden Suchen ist dieser Alternativansatz für eine einzelne Suche nicht so schnell wie das ursprünglich geschilderte Verfahren. Im Zusammenhang mit der Clusterung bedeutet dies jedoch keinen Zeitverlust. Da der Sequenzraum um die Anfangssequenz herum jedoch gründlicher durchsucht wird, umfaßt das resultierende Cluster mit großer Wahrscheinlichkeit bereits alle zu dieser Proteinfamilie gehörenden Sequenzen.

Als Datenbanksuchprogramm ist das Programm "BLASTP" sehr geeignet. Dieses Programm ist in Altschul, S. F., Gish, W., Miller, W. Myers, E. W. and Lipman, D. J., "Basic Local Alignment Search Tool", J. Mol. Biol., 215:403-410, 1990, näher beschrieben.

HARRIS N L ET AL: "ClassX: a browsing tool for protein sequence megaclassifications" PROCEEDING OF THE TWENTY-SIXTH HAWAII INTERNATIONAL CONFERENCE ON SYSTEM SCIENCES (CAT. NO.93TH0501-7), WAILEA, HI, USA, 5-8 JAN. 1993, Seiten 554-563 vol.1, XP002091600 ISBN 0-8186-3230-5, 1993, Los Alamitos, CA, USA, IEEE, USA, basiert auf das "BLAST" Programm. Alle ähnlichen Sequenzen zur gesuchten Sequenz werden paarweise recherchiert, diejenigen die genügend überlappen werden zusammen gruppiert und die so geformte Clusters werden verglichen.

Als Alternative kann jedoch auch das Datenbanksuchprogramm "FASTA" verwendet werden, das beispielsweise in der folgenden Literaturstelle beschrieben ist: Pearson, W. R. and Lipman, D. J., "Improved tools for biological sequence comparison", Proc. Natl. Acad. Sci. USA, 85:2444-2448, 1988.

Neben den Datenbanksuchprogrammen "BLASTP" und "FASTA" kann auch ein beliebiges anderes Datenbanksuchprogramm verwendet werden. Beispielsweise eigent sich auch das Programm "gapped-BLAST" (beschrieben in: Altschul, S.F., Madden, T.L., Schaeffer, A.A., Zhang, J., Zhang, Z., Miller, W. and Lipman, D.J., "Gapped BLAST and PSI-BLAST: a new generation of protein database search programs", Nucleic Acids Research, 25 (17):3389-3402, 1997)

Unter Verwendung des Programms "BLASTP" wurde die PIR1, Release 51 mengentheoretisch geclustert. Diese Datenbank enthält 13.489 Proteinsequenzen und ist näher beschrieben in David G. George, Richard J. Dodson, John S. Garavelli, Daniel H. Haft, Lois T. Hunt, Christopher R. Marzee, Bruce C. Orcutt, Kathryn E. Sidman, Geetha Y. Srinivasarao, Lai-Su L. Yeh, Lieslie M. Arminski, Robert S. Ledley, Akira Tsugita and Winona Barker. The protein information resource (PIR) and the PIR-international protein sequence database. Nucleic Acids Research, 25(1):24-27, 1997. Um diese Datenbank zu gruppieren, bedurfte es etwa eines Tages Rechenzeit und 91 % der Datenbanksequenzen wurden vollautomatisch in disjunkte Cluster eingruppiert. Das Residuum umfaßt nur ca. 9 % der Datenbanksequenzen.

Eine weit größere Datenbank ist die SWISS-PROT Datenbank, deren 34. Release 59.021 Sequenzen enthält. Diese Datenbank ist in Amos Bairoch and Rolf Apweiler. The SWISS-PROT protein sequence data bank and its supplement TrEMBL. Nucleic Acids Research, 25 (1):31-36, 1997 näher beschrieben. Hier wurden innerhalb von etwa 5 Tagen Rechenzeit 80 % der Sequenzen in disjunkte Klassen eingeordnet.

Diese Beispiele zeigen, daß gegenüber herkömmlichen Verfahren eine extreme Rechenzeitersparnis bei hervorragendem Clusterergebnis mit dem erfindungsgemäßen Verfahren zu erzielen ist.

Ein Algorithmus für das beschriebene Verfahren ist im folgenden dargestellt:

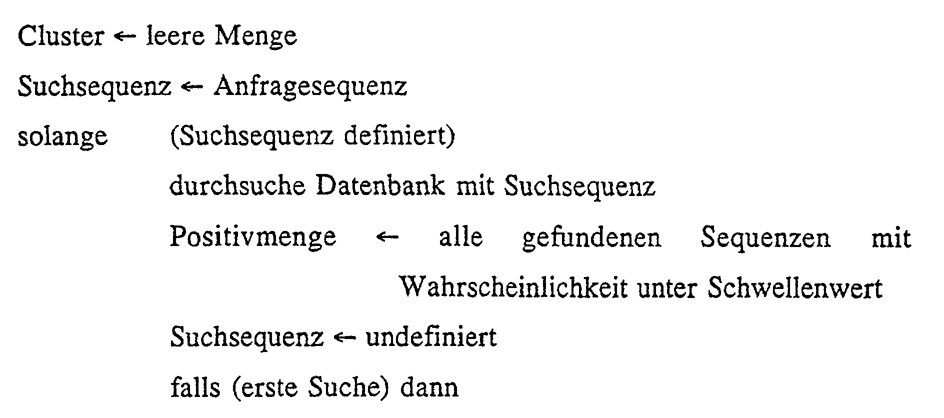

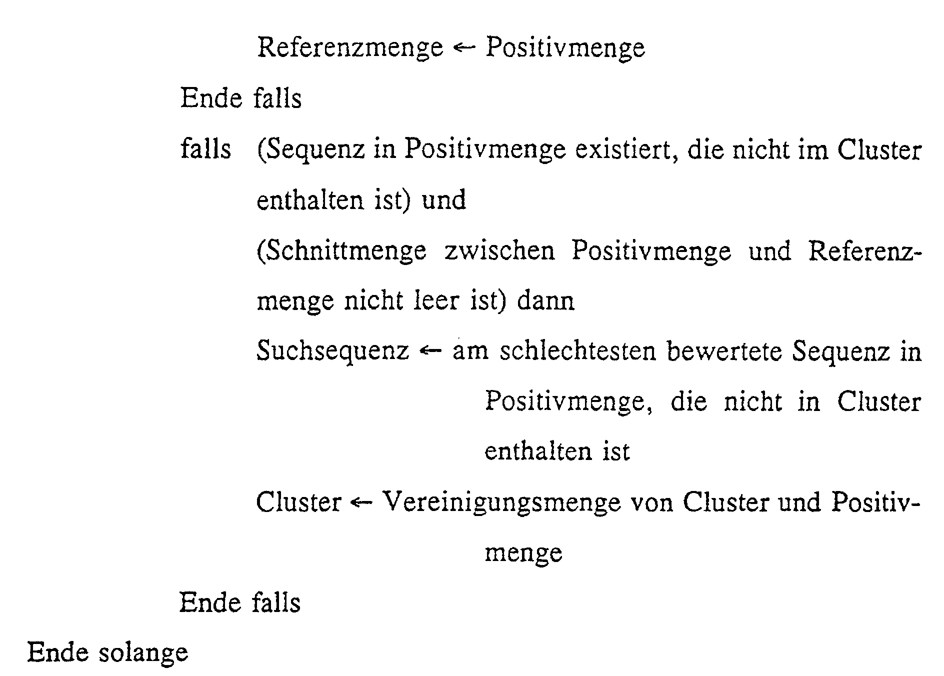

Die Sequenz mit der das Suchverfahren startet, wird Anfangssequenz genannt und die Menge der gefundenen Sequenzen, das zu dieser Anfangssequenz gehörige Cluster. Zunächst wird mit der Anfangssequenz ein Datenbanksuchprogramm zum Beispiel "BLASTP" oder "FASTA" gestartet und alle Sequenzen aus der Datenbank, die der Anfangssequenz signifikant ähnlich sind, werden akzeptiert. Diese Menge von Sequenzen nennen wir Positivmenge und nehmen sie als verwandte Sequenzen in das Cluster auf. Eine signifikante Ähnlichkeit zwischen zwei Sequenzen liegt dann vor, wenn die Wahrscheinlichkeit, daß diese Ähnlichkeit zufällig auftritt sehr gering ist, d. h. unterhalb eines gegebenen Schwellwertes liegt. Aus der erhaltenen Positivmenge verwenden wir nun die am schlechtesten bewertete Sequenz (d. h. die mit der höchsten Wahrscheinlichkeit) als Suchsequenz für eine weitere Datenbanksuche. Dieses Verfahren wird solange wiederholt, wie Sequenzen unterhalb des Schwellwertes gefunden werden, die noch nicht im Cluster enthalten sind, und wie es eine Schnittmenge zwischen der Positivmenge der Anfragesequenz und der Positivmenge der aktuellen Suchsequenz gibt.

Anschließend wird der folgende Algorithmus durchgeführt:

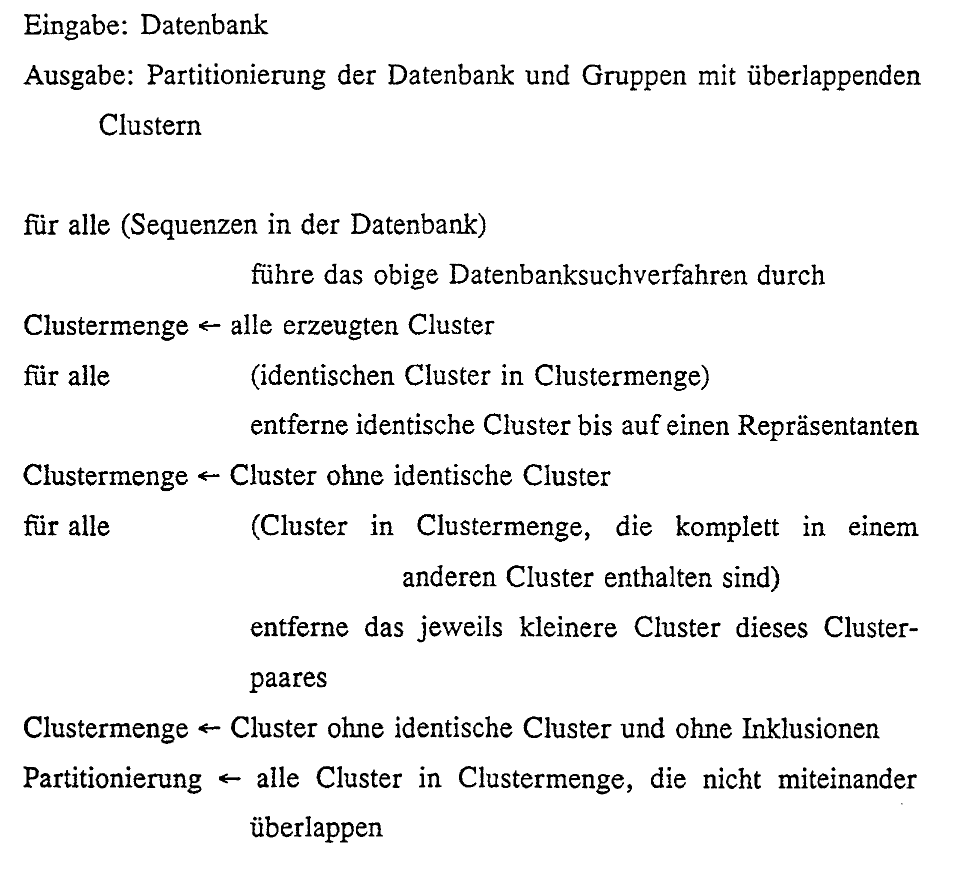

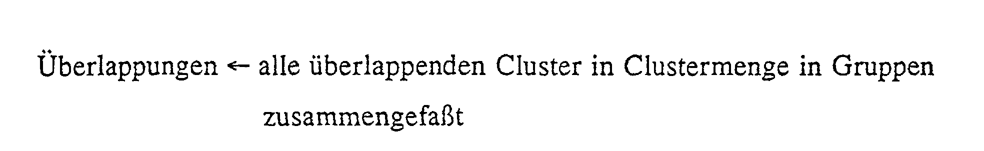

Um nun eine Datenbankclusterung zu erhalten, wird das zuvor beschriebene Verfahren für alle Sequenzen in der Datenbank durchgerührt, d. h. jeder Sequenz wird ein Cluster der mit ihr verwandten Sequenzen zugeordnet. Aus dieser Menge von Clustern werden nun jeweils bis auf ein Exemplar alle identischen Cluster entfernt, da sie keine zusätzlichen Informationen enthalten. Die verbleibende Clustermenge wird anschließend auf Inklusionen untersucht und Cluster, die komplett in anderen Clustern enthalten sind, entfernt, solange bis keine Inklusionen mehr vorhanden sind. Von dieser Clustermenge können nun diejenigen Cluster, die nicht mit anderen überlappen, als sinnvolle Partitionierung der Datenbank angesehen werden. Der verbleibende, geringe Teil an überlappenden Clustern wird in Gruppen zusammengefaßt, deren Cluster untereinander durch Überlappungen verbunden sind.

Ein Ausführungsbeispiel der Erfindung wird im folgenden näher beschrieben.

Als Anfragesequenz verwenden wir die Sequenz des menschlichen Homeobox Engrailed-1 Proteins (HME1_HUMAN) und durchsuchen damit die Swissprot Datenbank (Release 34) nach verwandten Sequenzen. Die Suche wird mit blastp durchgeführt und den Schwellwert wählen wir bei einer Wahrscheinlichkeit von 10⁻³⁰. Das Ergebnis dieser Suche sieht (auszugsweise) wie folgt aus:

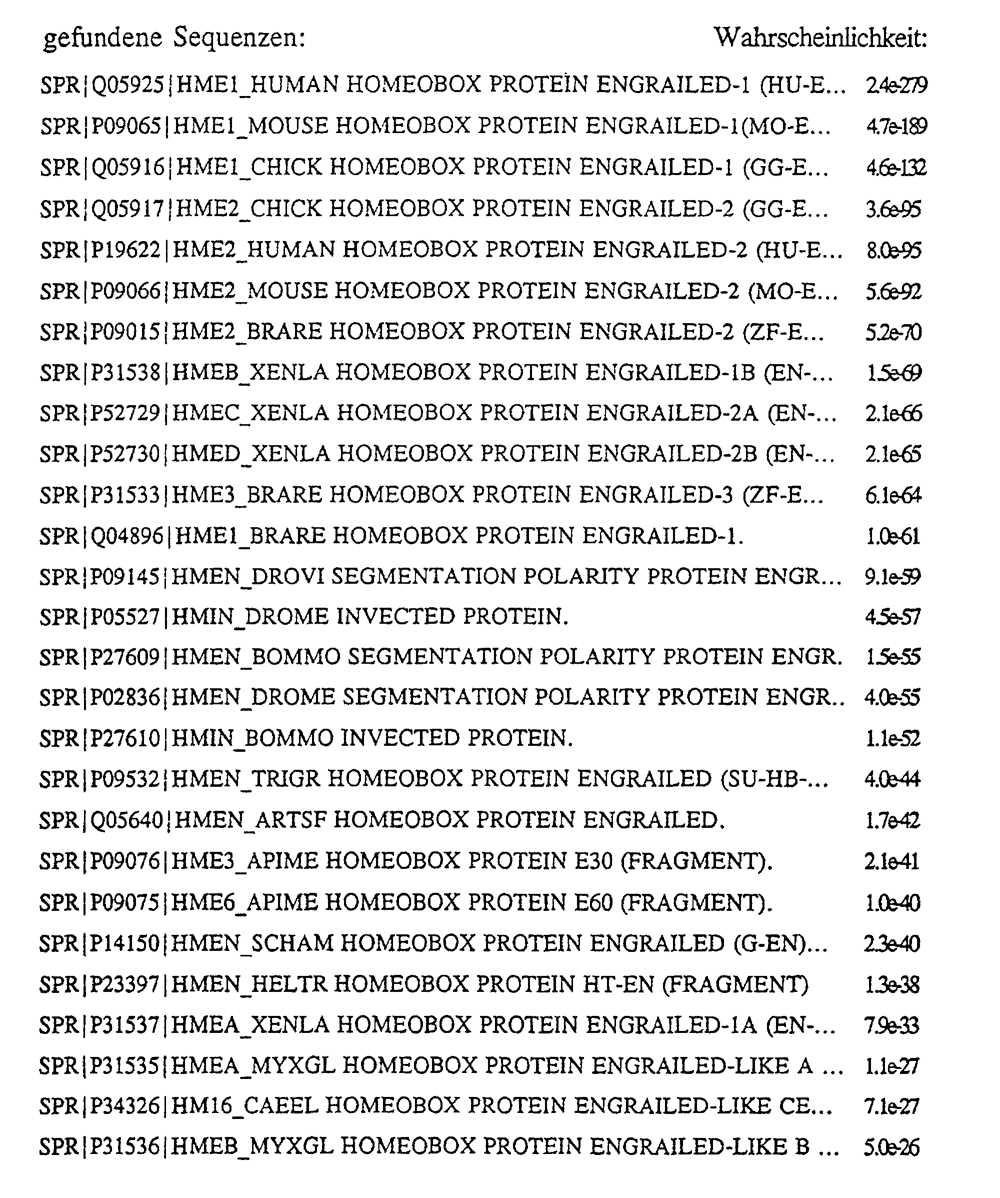

Aufgrund des Schwellwertes bei 10⁻³⁰ enthält unser Cluster nun die Sequenzen:

HME1_HUMAN, HME1_MOUSE, HME1_CHICK, HME2_CHICK,
HME2_HUMAN, HME2_MOUSE, HME2_BRARE, HMEB_XENLA,
HMEC_XENLA, HMED_XENLA, HME3_BRARE, HME1_BRARE,
HMEN_DROVI, HMIN_DROME, HMEN_BOMMO, HMEN_DROME,
HMIN_BOMMO, HMEN_TRIGR, HMEN_ARTSF, HME3_APIME,
HME6_APIME, HMEN_SCHAM, HMEN_HELTR, HMEA_XENLA.

Der nächste Durchlauf des blastp-Programms wird nun mit der am schlechtesten bewerteten Sequenz in dieser Menge durchgeführt nämlich mit dem Homeobox Engrailed-1A Protein des Krallenfrosches (HMEA_XENLA). Das Ergebnis dieser Suche sieht (auszugsweise) wie folgt aus:

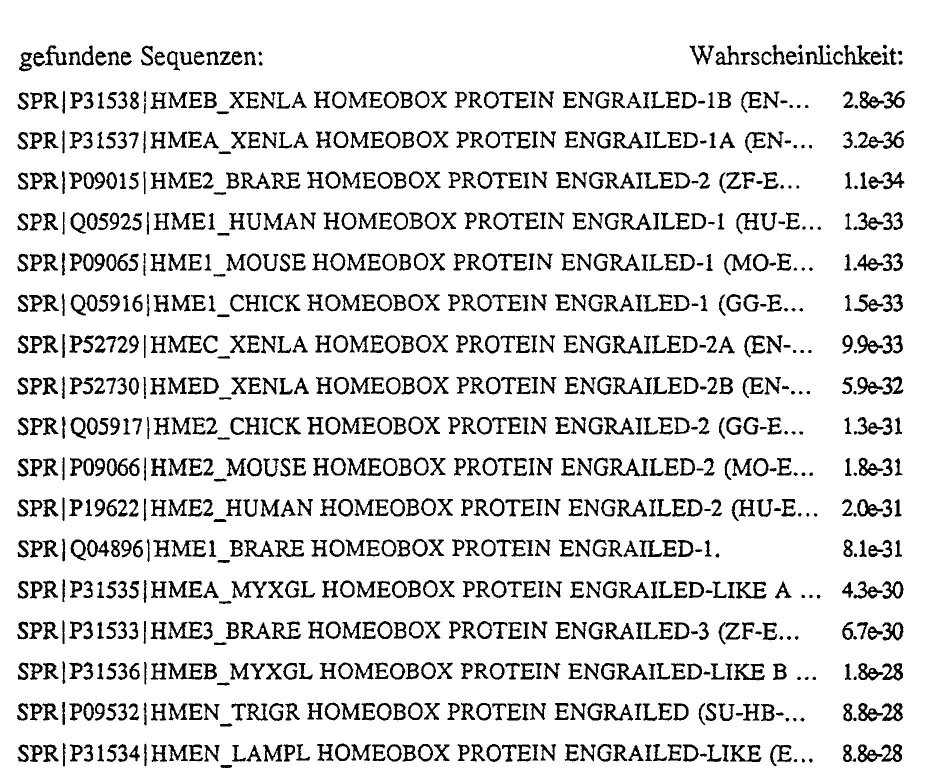

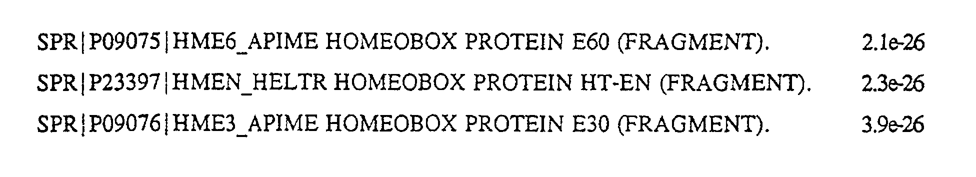

Wieder betrachten wir alle Sequenzen mit einer niedrigeren Wahrscheinlichkeit als 10⁻³⁰ und stellen fest, daß bis auf HMEA_MYXGL bereits alle Sequenzen im Cluster enthalten sind. Diese Sequenz wird nun in das Cluster aufgenommen und mit ihr wird die nächste blastp-Suche gestartet. Das Ergebnis dieser Suche sieht (auszugsweise) wie folgt aus:

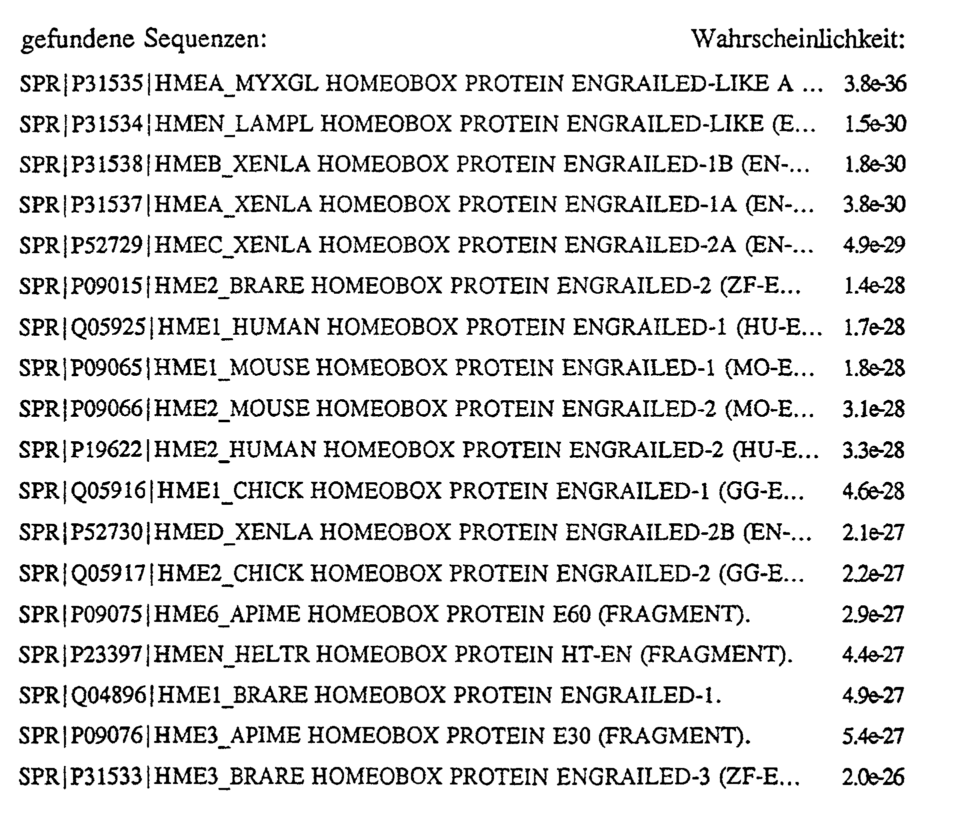

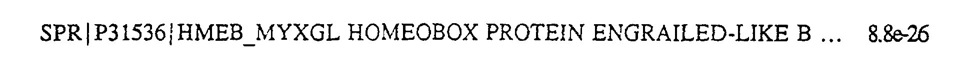

Diesmal fügen wir noch HMEN_LAMPL zu unserem Cluster hinzu und starten mit dieser Sequenz die nächste blastp-Suche, die (auszugsweise) das folgende Ergebnis liefert:

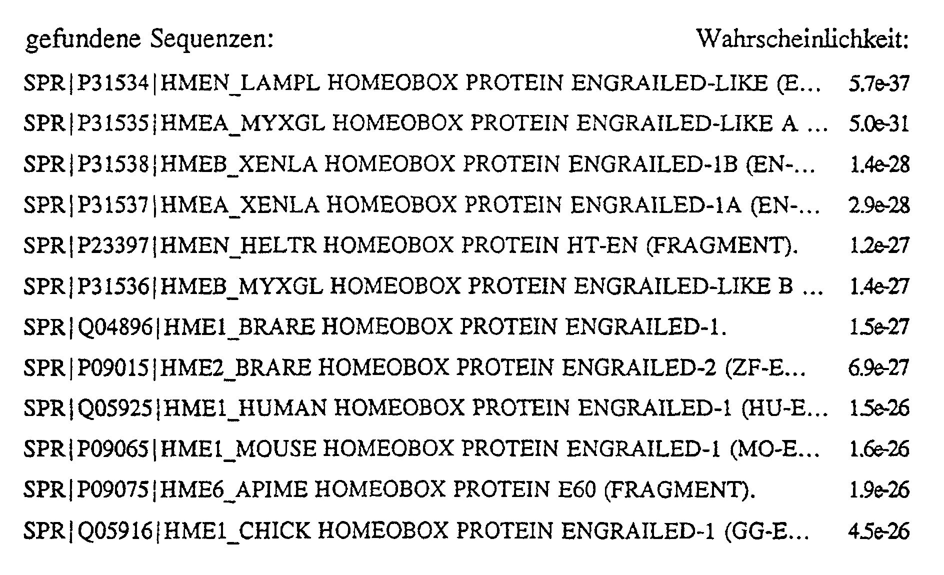

Oberhalb des Schwellwertes finden wir nun keine Sequenzen mehr, die nicht schon in unserem Cluster enthalten wären, so daß die SYSTERS-Suche für diese Anfragesequenz nun beendet wird und das Cluster die folgenden 26 Sequenzen enthält:

HME1_HUMAN, HME1_MOUSE, HME1_CHICK, HME2_CHICK,

HME2_HUMAN, HME2_MOUSE, HME2_BRARE, HMEB_XENLA,
HMEC_XENLA, HMED_XENLA, HME3_BRARE, HME1_BRARE,
HMEN_DROVI, HMIN_DROME, HMEN_BOMMO, HMEN_DROME,
HMIN_BOMMO, HMEN_TRIGR, HMEN_ARTSF, HME3_APIME,
HME6_APIME, HMEN_SCHAM, HMEN_HELTR, HMEA_XENLA,
HMEA_MYXGL, HMEN_LAMPL.

Führt man dieses Verfahren für alle 28 in der Swissprot Datenbank mit "Homeobox Engrailed" annotierten Sequenzen durch, so erhält man zunächst 28 Cluster. In der folgenden Tabelle sind die gefundenen Cluster gegen die Sequenzen aufgetragen, wobei eine Spalte das zu der im Kopf angegebenen Anfragesequenz gehörige Cluster repräsentiert und eine Zeile die Cluster angibt, in der die links aufgeführte Sequenz enthalten ist (markiert durch ein X). In diesem Fall gibt es sieben Cluster mit jeweils 27 Sequenzen, fünf Cluster mit jeweils 26 Sequenzen usw.

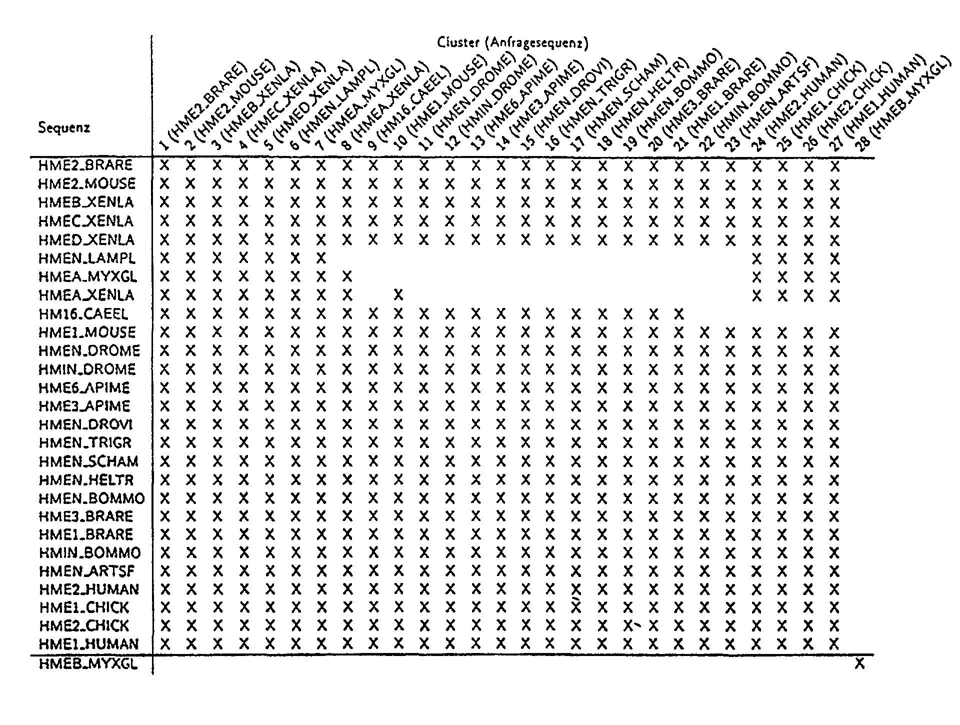

Nach dem Entfernen identischer Cluster und dem Auflösen von Inklusionen verteilen sich die Homeobox Engrailed Proteine auf 2 Cluster - das eine mit 27 Sequenzen, das andere lediglich mit der Sequenz HMEB_MYXGL.

## Patentansprüche

1. Verfahren zur Eingruppierung von Sequenzen in Familien, bei dem
- mit einem Datenbanksuchprogramm aus einer Sequenzdatenbank als Positivmenge alle zu einer Anfragesequenz ähnlichen Sequenzen ermittelt werden, für die die Wahrscheinlichkeit, daß die Ähnlichkeit zufällig auftritt, unterhalb eines vorgegebenen Schwellenwertes liegt,
- aus dieser Positivmenge mindestens eine Sequenz als Suchsequenz ausgewählt wird,
- anschließend das beschriebene Suchverfahren jeweils mit der ermittelten Suchsequenz als Anfragesequenz solange wiederholt wird, wie die gerade ermittelte Positivmenge Sequenzen enthält, die in den zuvor ermittelten Positivmengen nicht enthalten sind und es eine Schnittmenge zwischen der Positivmenge der Anfragesequenz und der Positivmenge der aktuellen Suchsequenz gibt und
- alle in den berechneten Positivmengen enthaltenen unterschiedlichen Sequenzen als Cluster ausgegeben werden.

2. Verfahren nach Anspruch 1, ***dadurch gekennzeichnet, daß***
- das Verfahren nach Anspruch 1 für jede Sequenz der Datenbank durchgerührt wird,
- die mehrfach auftretenden Cluster bis auf jeweils einen Cluster entfernt werden,
- Cluster, die in anderen Clustern enthalten sind, entfernt werden,
- von der verbleibenden Clustermenge die Cluster, die nicht mit anderen Clustern überlappen als Partitionierung der Datenbank ausgegeben werden und
- der verbleibende Teil der überlappenden Cluster als Gruppen ausgegeben wird, deren Cluster untereinander durch Überlappungen verbunden sind.

3. Verfahren nach einem der vorhergehenden Ansprüche, ***dadurch gekennzeichnet, daß*** der Schwellenwert zwischen 10⁻²⁰ bis 10⁻³⁵ liegt.

4. Verfahren nach einem der vorhergehenden Ansprüche, ***dadurch gekennzeichnet, daß*** als Positivmenge die Sequenz mit der höchsten Wahrscheinlichkeit ausgewählt wird.

5. Verfahren nach einem der Ansprüche 1 bis 3, ***dadurch gekennzeichnet, daß*** alle Sequenzen der Positivmenge als Suchsequenz dienen.

6. Verfahren nach einem der vorhergehenden Ansprüche, ***dadurch gekennzeichnet, daß*** als Datenbanksuchprogramm das Programm "BLASTP" verwendet wird.

7. Verfahren nach einem der Ansprüche 1 bis 3, ***dadurch gekennzeichnet, daß*** als Datenbanksuchprogramm das Programm "FASTA" verwendet wird.

8. Verfahren nach einem der Ansprüche 1 bis 3, ***dadurch gekennzeichnet, daß*** als Datenbanksuchprogramm das Programm "gapped-BLAST" verwendet wird.

## Claims

1. A method for classifying sequences into families, wherein
- using a database search program, all sequences similar to a request sequence, for which the probability that the similarity is coincidental lies below a predetermined threshold value, are determined from a sequence database as a positive quantity,
- from this positive quantity, at least one sequence is selected as a search sequence,
- the search process described is then repeated with each of the search sequences determined as the request sequence as long as the positive quantity just detected contains sequences which were not contained in the previously detected positive quantities and there is an intersection between the positive quantity of the request sequence and the positive quantity of the current search sequence, and
- all of the different sequences contained in the calculated positive quantities are output as a cluster.

2. The process according to claim 1, ***characterized in that***
- the process according to claim 1 is performed for each sequence of the databank,
- multiply occurring clusters are removed except for one cluster,
- clusters which are contained in other clusters are removed,
- from the remaining cluster quantity, the clusters which do not overlap with other clusters are output as a partitioning of the database, and
- the remaining portion of the overlapping clusters are output as groups whose clusters are connected to one another by overlaps.

3. The process according to one of the preceding claims, ***characterized in that*** the threshold value lies between 10⁻²⁰ to 10⁻³⁵.

4. The process according to one of the preceding claims, ***characterized in that*** the sequence with the highest probability is selected as the positive quantity.

5. The process according to one of the claims 1 to 3, ***characterized in that*** all sequences of the positive quantity serve as search sequences.

6. The process according to one of the preceding claims, ***characterized in that*** the program "BLASTP" is used as the database search program.

7. The process according to one of the claims 1 to 3, ***characterized in that*** the program "FASTA" is used as the database search program.

8. The process according to one of the claims 1 to 3, ***characterized in that*** the program "gapped-BLAST" is used as the database search program.

## Revendications

1. Procédé de regroupement de séquences en famille, dans lequel
- toutes les séquences similaires à une séquence de requête sont déterminées en tant qu'ensemble positif avec un programme de recherche dans une base de données, à partir d'une base de données de séquences, pour lesquelles la probabilité que la similitude se produise aléatoirement est inférieure à une valeur de seuil prédéfinie,
- au moins une séquence est sélectionnée en tant que séquence de recherche à partir de cet ensemble positif,
- ensuite, le procédé de recherche décrit est répété respectivement avec la séquence de recherche déterminée en tant que séquence de requête, jusqu'à ce que l'ensemble positif qui vient d'être déterminée contienne des séquences, qui ne sont pas contenues dans les ensembles positifs déterminés précédemment et il existe un ensemble d'intersections entre l'ensemble positif de la séquence de requête et l'ensemble positif de la séquence de recherche actuelle et
- toutes les séquences différentes contenues dans les ensembles positifs calculés sont sorties sous forme de grappes.

2. Procédé selon la revendication 1, ***caractérisé en ce que,***
- le procédé, selon la revendication 1, est réalisé pour chaque séquence de la base de données,
- les grappes survenant de façon multiple sont éliminés jusqu'à l'obtention respective d'une grappe,
- les grappes, qui sont contenues dans d'autres grappes, sont éliminées,
- parmi l'ensemble des grappes restantes, les grappes qui ne recouvrent pas les autres grappes sont sorties en tant que partitionnement de la base de données et
- la partie restante des grappes à recouvrement est sortie sous forme de groupes dont les grappes sont liées entre elles par des recouvrements.

3. Procédé selon l'une quelconque des revendications précédentes ***caractérisé en ce que*** la valeur de seuil est comprise entre 10⁻²⁰ à 10⁻³⁵.

4. Procédé selon l'une quelconque des revendications précédentes, ***caractérisé en ce que*** l'on choisit la séquence ayant la probabilité la plus élevée, comme ensemble positif.

5. Procédé selon l'une quelconque des revendications 1 à 3 ***caractérisé en ce que*** toutes les séquences de l'ensemble positif servent de séquence de recherche.

6. Procédé selon l'une quelconque des revendications précédentes, ***caractérisé en ce que*** l'on utilise le programme "BLASTP" comme programme de recherche dans la base données.

7. Procédé selon l'une quelconque des revendications 1 à 3, ***caractérisé en ce que*** l'on utilise le programme "FASTA" comme programme de recherche dans la base de données.

8. Procédé selon l'une quelconque des revendications 1 à 3, ***caractérisé en ce que,*** l'on utilise le programme "gapped-BLAST". comme programme de recherche dans la base de données.
